# EUROPEAN PATENT SPECIFICATION

(11) **EP 3 052 188 B1**
(45) Date of publication and mention of the grant of the patent: **01.12.2021**
(21) Application number: 14781047.7
(22) Date of filing: 30.09.2014
(51) Int. Cl.: A61N 5/10

(54) **PREDICTING ACHIEVABLE DOSE DISTRIBUTION USING 3D INFORMATION AS AN INPUT**
VORHERSAGE DER ERREICHBAREN DOSISVERTEILUNG MIT 3D-INFORMATIONEN ALS EINGABE
PRÉVISION DE DISTRIBUTION DE DOSE RÉALISABLE AU MOYEN D'INFORMATIONS 3D SERVANT D'ENTRÉE

(30) Priority: 30.09.2013 US 201361884363 P
(43) Date of publication of application: 10.08.2016
(73) Proprietor: Varian Medical Systems International AG, 6312 Steinhausen (CH)
(72) Inventor: KUUSELA, Esa, FI-02320 Espoo (FI); HARTMAN, Joona, FI-02200 Espoo (FI); NORD, Janne, FI-02620 Espoo (FI); BAGHAIE, Ramin, FI-02360 Espoo (FI)
(74) Representative: Foster, Mark Charles
(86) International application number: PCT/CH2014/000139
(87) International publication number: WO 2015/042727

(56) References cited:
- WO-A2-2012/024448
- US-A1- 2011 124 976
- BOMANJI J ET AL: "Clinical role of positron emission tomography in oncology", LANCET ONCOLOGY, LANCET PUBLISHING GROUP, LONDON, GB, vol. 2, no. 3, 1 March 2001 (2001-03-01), pages 157-164, XP004811668, ISSN: 1470-2045, DOI: 10.1016/S1470-2045(00)00257-6
- None

## Description

### BACKGROUND

The use of radiation therapy to treat cancer is well known. Typically, radiation therapy involves directing a beam of high energy proton, photon, or electron radiation ("therapeutic radiation") into a target volume (e.g., a tumor or lesion).

Before a patient is treated with radiation, a treatment plan specific to that patient is developed. The plan defines various aspects of the therapy using simulations and optimizations based on past experiences. For example, for intensity modulated radiation therapy (IMRT), the plan can specify the appropriate beam type (e.g., flattening filter free type) and the appropriate beam energy. Other parts of the plan can specify, for example, the angle of the beam relative to the patient, the beam shape, the placement of boluses and shields, and the like. In general, the purpose of the treatment plan is to deliver sufficient radiation to the target volume while minimizing exposure of surrounding healthy tissue to the radiation. Treatment plans are usually assessed with the aid of dose-volume histograms (DVHs) that, generally speaking, represent three-dimensional (3D) dose distributions in two dimensions. Document WO 2012/024448 A2 discloses an automated treatment planning for radiation therapy using an expert system which includes information on a plurality of patients tumor parameters used to treat the tumor.

### SUMMARY

The invention is as defined in the appended claims.

Knowledge-based planning (KBP) uses data from previous and existing radiation therapy/treatment plans to predict a clinically optimal or acceptable outcome for a new patient based on geometric information known about the patient. The geometric information, such as the contours of the patient's anatomical structures (e.g., organs), can be predefined information that is adapted to (e.g., registered to) the patient, or it can be based on, for example, computed tomography (CT) scans of the patient. A practical implementation of KBP includes a model training phase in which historical information from previous and existing treatment plans are used to generate a generic prediction model, followed by an estimation phase in which the generic model is used to determine (e.g., estimate or predict) an achievable dose distribution for a particular patient based on geometric information for that patient.

Images obtained using techniques such as, but not limited to, positron emission tomography (PET) can be used to provide information about the spatial distribution of biological or chemical activity. PET provides a known technique for diagnosing and monitoring cancer; it can be used to detect a cancerous tumor and the extent (e.g., size) of the tumor, and can be used to determine the tumor's response to treatment. The spatial information can be used to tune the desired dose level to the target structure during radiation treatment planning, a process known as dose painting. "Dose-painting" radiotherapy allows for a heterogeneous delivery of radiation within a target volume (e.g., within a tumor volume); it is possible to apply a strategy of intensity modulated radiation therapy (IMRT) that will deliver a higher dose to certain regions within the volume. In other words, instead of applying a uniform dose level to the entire target volume, the dose is redistributed so that, for example, some regions receive a higher dose.

In embodiments according to the present invention, knowledge-based planning is supplemented with spatially distributed (3D) biological or chemical activity information to expedite creation of treatment plans and to improve the quality of those plans. More specifically, the information used in the model training phase is expanded to include such spatial information. In an embodiment, KBP is extended to include spatial information from, for example, PET images. However, embodiments according to the present invention are not limited to PET images; other types of images, such as but not limited to functional magnetic resonance imaging (MRI) images, can be used. In general, embodiments according to the present invention can utilize KBP in combination with any 3D imaging technology that yields information about the composition of a target volume.

In an embodiment, information that is specific to a patient is accessed. A prediction of a spatial dose distribution inside a target volume in the patient is determined using the patient-specific information as an input to a prediction model. The prediction model is established using training data that includes data resulting from applying other radiation treatment plans to other patients. The training data includes spatially distributed information indicating a level of activity in target volumes in the other patients (e.g., PET image data). A dose-volume histogram and associated three-dimensional dose distribution information based on the prediction are produced. The dose-volume histogram and the three-dimensional dose distribution information can be used to develop a radiation treatment plan for the patient.

As a result, the quality of the treatment plans is improved with respect to, for example, the ability to deliver sufficient radiation to the target volume while minimizing exposure of surrounding healthy tissue to the radiation. The quality of the generic prediction model is also improved, facilitating the creating of treatment plans.

This summary is provided to introduce a selection of concepts in a simplified form that is further described below in the detailed description that follows. This summary is not intended to identify key features or essential features of the claimed subject matter, nor is it intended to be used to limit the scope of the claimed subject matter.

### BRIEF DESCRIPTION OF DRAWINGS

The accompanying drawings, which are incorporated in and form a part of this specification and in which like numerals depict like elements, illustrate embodiments of the present disclosure and, together with the detailed description, serve to explain the principles of the disclosure.
Figure 1 shows a block diagram of an example of a computing system upon which the embodiments described herein may be implemented.
Figure 2 shows a process that can be implemented to create and use a prediction model in an embodiment according to the present invention.
Figure 3 is a block diagram illustrating an example of an automated radiation therapy treatment planning system in an embodiment according to the present invention.
Figure 4 illustrates an embodiment of a knowledge-based planning system in an embodiment according to the present invention.
Figure 5 is a flowchart of an example of a computer-implemented method for generating a radiation therapy treatment plan in an embodiment according to the present invention.

### DETAILED DESCRIPTION

Reference will now be made in detail to the various embodiments of the present disclosure, examples of which are illustrated in the accompanying drawings. While described in conjunction with these embodiments, it will be understood that they are not intended to limit the disclosure to these embodiments. On the contrary, the disclosure is intended to cover alternatives, modifications and equivalents. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

Some portions of the detailed descriptions that follow are presented in terms of procedures, logic blocks, processing, and other symbolic representations of operations on data bits within a computer memory. These descriptions and representations are the means used by those skilled in the data processing arts to most effectively convey the substance of their work to others skilled in the art. In the present application, a procedure, logic block, process, or the like, is conceived to be a self-consistent sequence of steps or instructions leading to a desired result. The steps are those utilizing physical manipulations of physical quantities. Usually, although not necessarily, these quantities take the form of electrical or magnetic signals capable of being stored, transferred, combined, compared, and otherwise manipulated in a computing system. It has proven convenient at times, principally for reasons of common usage, to refer to these signals as transactions, bits, values, elements, symbols, characters, samples, pixels, or the like.

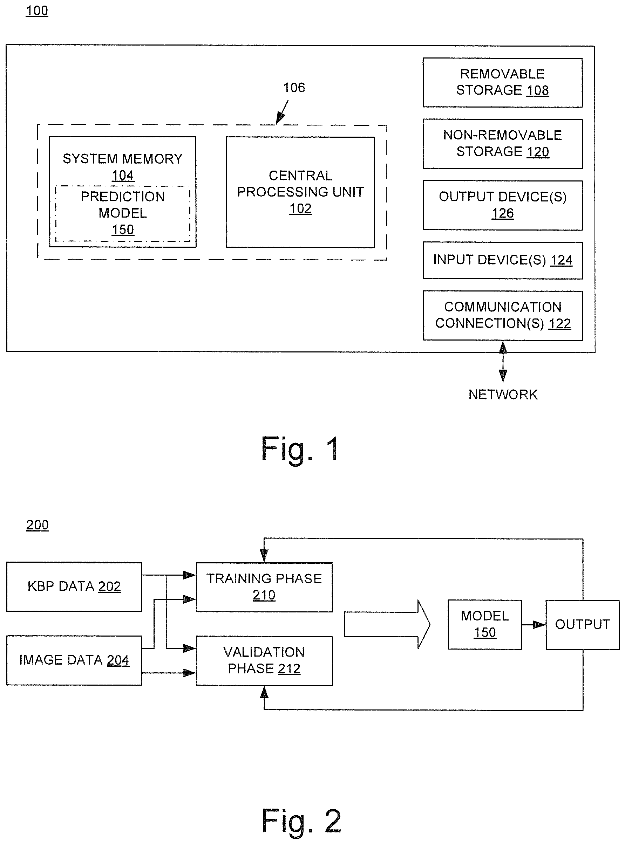

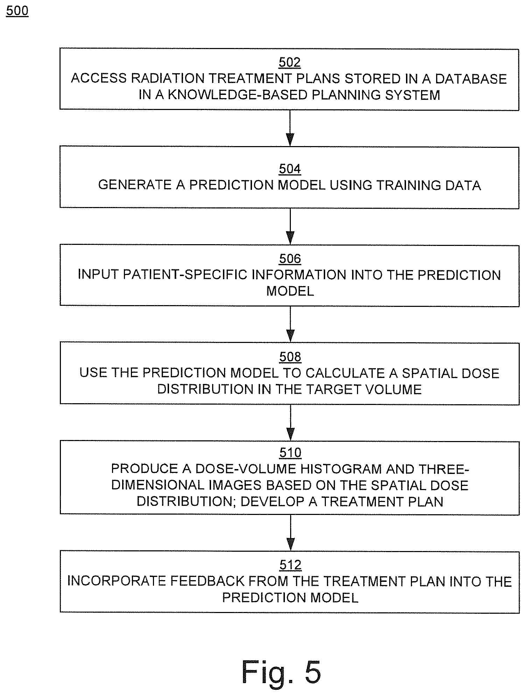

It should be borne in mind, however, that all of these and similar terms are to be associated with the appropriate physical quantities and are merely convenient labels applied to these quantities. Unless specifically stated otherwise as apparent from the following discussions, it is appreciated that throughout the present disclosure, discussions utilizing terms such as "determining," "accessing," "producing," "using," "inputting," "calculating," "generating", or the like, refer to actions and processes (e.g., the flowchart 500 of Figure 5) of a computing system or similar electronic computing device or processor (e.g., the computing system 100 of Figure 1). The computing system or similar electronic computing device manipulates and transforms data represented as physical (electronic) quantities within the computing system memories, registers or other such information storage, transmission or display devices.

Portions of the detailed description that follows are presented and discussed in terms of a method. Although steps and sequencing thereof are disclosed in figures herein (e.g., Figure 5) describing the operations of this method, such steps and sequencing are exemplary. Embodiments are well suited to performing various other steps or variations of the steps recited in the flowchart of the figure herein, and in a sequence other than that depicted and described herein.

Embodiments described herein may be discussed in the general context of computer-executable instructions residing on some form of computer-readable storage medium, such as program modules, executed by one or more computers or other devices. By way of example, and not limitation, computer-readable storage media may comprise non-transitory computer storage media and communication media. Generally, program modules include routines, programs, objects, components, data structures, etc., that perform particular tasks or implement particular abstract data types. The functionality of the program modules may be combined or distributed as desired in various embodiments.

Computer storage media includes volatile and nonvolatile, removable and non-removable media implemented in any method or technology for storage of information such as computer-readable instructions, data structures, program modules or other data. Computer storage media includes, but is not limited to, random access memory (RAM), read only memory (ROM), electrically erasable programmable ROM (EEPROM), flash memory or other memory technology, compact disk ROM (CD-ROM), digital versatile disks (DVDs) or other optical storage, magnetic cassettes, magnetic tape, magnetic disk storage or other magnetic storage devices, or any other medium that can be used to store the desired information and that can accessed to retrieve that information.

Communication media can embody computer-executable instructions, data structures, and program modules, and includes any information delivery media. By way of example, and not limitation, communication media includes wired media such as a wired network or direct-wired connection, and wireless media such as acoustic, radio frequency (RF), infrared and other wireless media. Combinations of any of the above can also be included within the scope of computer-readable media.

Figure 1 shows a block diagram of an example of a computing system 100 upon which the embodiments described herein may be implemented. In its most basic configuration, the system 100 includes at least one processing unit 102 and memory 104. This most basic configuration is illustrated in Figure 1 by dashed line 106. The system 100 may also have additional features/functionality. For example, the system 100 may also include additional storage (removable and/or non-removable) including, but not limited to, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 may also have input device(s) 124 such as keyboard, mouse, pen, voice input device, touch input device, etc. Output device(s) 126 such as a display, speakers, printer, etc., may also be included.

In the example of Figure 1, the memory 104 includes computer-readable instructions, data structures, program modules and the like associated with a prediction model 150. However, the prediction model 150 may instead reside in any one of the computer storage media used by the system 100, or may be distributed over some combination of the computer storage media, or may be distributed over some combination of networked computers.

Figure 2 shows a process 200 that can be implemented to create and use the prediction model 150 in an embodiment according to the present invention. Process 200 can be implemented as computer-readable instructions stored in a computer-usable medium.

Knowledge based planning (KBP) data 202 includes data from previous and existing radiation therapy plans to predict a clinically optimal or acceptable outcome for a new patient based on geometric information known about the patient.

Image data 204 includes data obtained using techniques can be used to provide information about the composition of a target volume (e.g., tumor) based on, for example, the spatial distribution of biological or chemical activity in the target volume. Such techniques include, but are not limited to, positron emission tomography (PET) and functional magnetic resonance imaging (MRI).

Embodiments according to the present invention extend KBP to include the spatial information from, for example, PET images. More specifically, the information used in the model training phase 210 is expanded to include such spatial information. Such information can also be used in a model validation phase 212.

In the model training phase 210, the prediction model 150 is developed and trained using training data. The training data is based on a first sample of different treatment plans developed for various patients; the training data may include, for example, dose-volume histograms (DVHs) for the first sample of treatment plans. The treatment plans in the model training phase 210 are developed using spatial information (e.g., PET images). In the model validation phase 212, the prediction model 150 is evaluated based on its performance on the training data (its ability to accurately model the training data and the DVHs for the training data) as well as its ability to satisfactorily predict validation data based on another (second) sample of treatment plans (its ability to accurately model the validation data and the DVHs for the validation data). The adequacy of the prediction model 150 is demonstrated by its capability to satisfactorily model and predict both the training data and the validation data.

The KBP data 202 and the image data 204 can be used in the model training phase 210 to create the prediction model 150 for spatial dose distribution inside targets to reflect the spatial dose distribution variation from dose painting. In an embodiment, a regression model between image pixel intensity and dose that would be used spatially can be found - the relationship between, for example, PET spatial information and variation in target dose levels can be found. In another embodiment, other structural information, such as the distance from the edge of a target structure, can be used to determine the spatial dose distribution. The prediction model 150 can also be based on certain image processing steps (e.g., smoothing of PET images) or other similar manipulations.

Training data included in the model training phase 210 can be appropriately considered and assessed using the regression model, for example, until the prediction model 150 is produced. Once the training data can be satisfactorily predicted using the model 150, then the data included in the validation phase 212 can be used to independently test and verify the accuracy of the model. Model development is an iterative process between training and validation that proceeds until the validation data is satisfactorily predicted.

The prediction model 150 can be updated as needed. As experience is gained using the model, results can be fed back into the training data or the validation data to continually refine the prediction model.

Clinicians can select their "best" treatment plans to include in a training set that can be used to improve the model 150 and/or create a new model. The model 150 can be shared across the healthcare network to create a standard and more comprehensive model.

In this manner, a prediction model (the model 150) that includes KBP assisted by 3D images (e.g., PET images) can be used to create automatically dose painting objectives as part of a radiotherapy treatment plan. The prediction model 150 can be used to develop a treatment plan for a new patient by estimating an achievable dose distribution in a target volume (e.g., a tumor) inside the patient. The estimate can be made by inputting patient-specific information (e.g., geometry information) into the prediction model 150, which can predict a dose distribution based on the model's distillation of the results of the treatment plans used in the training data and/or validation data.

The input patient-specific information may contain any combination of parameters that can practically affect the radiation therapy treatment plan. For example, the patient-specific information may be organized as a vector or a data structure including feature elements for: size and shape of the target volume; location of the target volume; size and shape of an organ at risk; type of an organ at risk; a part of the target volume that overlaps an organ; and a part of an organ that overlaps the target volume.

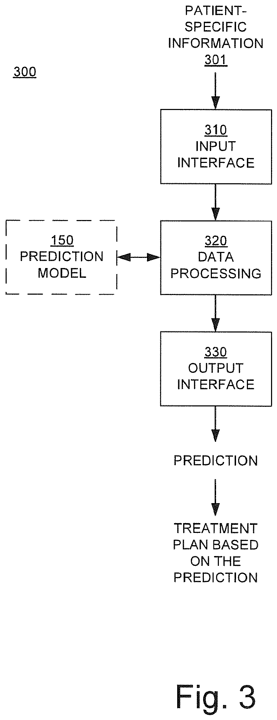

Figure 3 is a block diagram illustrating an example of an automated radiation therapy treatment planning system 300 in an embodiment according to the present invention. The system 300 includes an input interface 310 to receive patient-specific information (data) 301, a data processing component 320 that implements the prediction model 150, and an output interface 330. The system 300 in whole or in part may be implemented as a software program, hardware logic, or a combination thereof on/using the computing system 100 (Figure 1).

The patient-specific information is provided to and processed by the prediction model 150. The prediction model 150 yields a prediction result, e.g., an achievable dose distribution prediction. A treatment plan based on the prediction result can then be generated. In an embodiment, the prediction result is accompanied by parameters indicative of the quality of the prediction, such as reliability of the result (e.g., affected by the internal coherence of the training data), complexity of the predicted plan, and probability of the result.

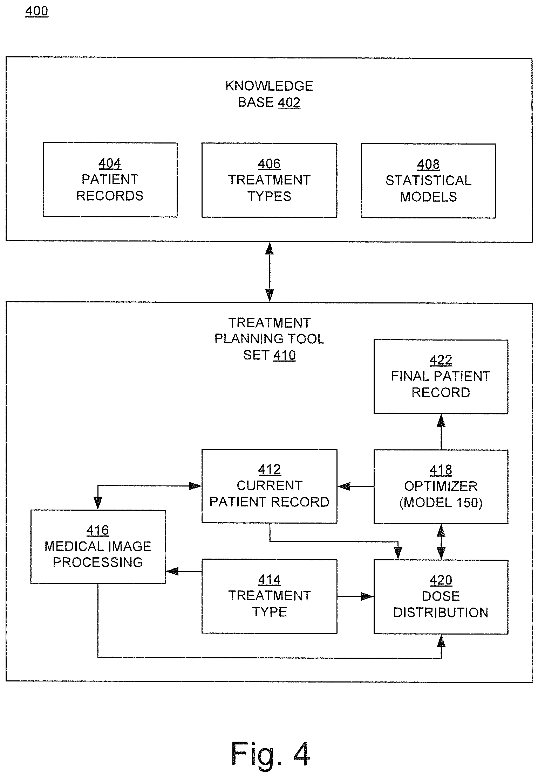

Figure 4 illustrates an embodiment of a knowledge-based planning system 400 incorporating a combination of patient records and statistical models to simplify the generation of radiation therapy dose distribution treatment plans, in an embodiment according to the present invention. In the example of Figure 4, the system 400 includes a knowledge base 402 and a treatment planning tool set 410. The knowledge base 402 includes patient records 404 (e.g. radiation treatment plans), treatment types 406, and statistical models 408. The treatment planning tool set 410 in the example of Figure 4 includes a current patient record 412, a treatment type 414, a medical image processing module 416, an optimizer 418, a dose distribution module 420, and a completed patient record 422 (e.g., a final, approved treatment plan).

The treatment planning tool set 410 searches through the knowledge base 402 (through the patient records 404) for prior patient records that are similar to the current patient record 412. The statistical models 408 can be used to compare the current treatment plan's predicted results to a statistical patient. Using the current patient information, a selected treatment type 406, and selected statistical models 408, the tool set 410 generates an optimized treatment plan that can be then approved by a clinician.

Based on past clinical experience, when a patient presents with a particular diagnosis, stage, age, weight, sex, co-morbidities, etc., there can be a treatment type that is used most often. By selecting the treatment type that the clinician has used in the past for similar patients, a first-step treatment type 414 can be chosen. The medical image processing module 416 provides automatic contouring and automatic segmentation of two-dimensional cross-sectional slides (e.g., from CT or MRI) to form a three-dimensional image with the medical images in the current patient record 412. Dose distribution maps are calculated by the dose distribution module 420.

The knowledge base 402 can be searched for a combination of objectives that can be applied by the optimizer 418 to determine a dose distribution. For example, an average organ-at-risk dose-volume histogram, a mean cohort organ-at-risk dose-volume histogram, and average organ-at-risk objectives can be selected from the knowledge base 402. In embodiments according to the present invention, the optimizer 418 uses the prediction model 150 to help shape the dose distribution. Accordingly, the optimizer 418 can provide a three-dimensional dose distribution, fluences, and associated dose-volume histograms for the current patient. By using the prediction model 150, which is trained and validated based on historical data as described above, those results are expected to fall within the historically accepted range for a patient with a similar disease type and treatment type.

Figure 5 is a flowchart 500 of an example of a computer-implemented method for generating a radiation therapy treatment plan in an embodiment according to the present invention. The flowchart 500 can be implemented as computer-executable instructions residing on some form of computer-readable storage medium (e.g., using computing system 100 of Figure 1).

In block 502 of Figure 5, radiation treatment plans that are stored in a database in a knowledge-based planning system are accessed. The radiation treatment plans were developed using spatially distributed information (e.g., PET image data) indicating levels of activity in target volumes in the patients associated with the treatment plans.

In block 504, a prediction model is generated using training data that includes the results from applying the radiation treatment plans to the patients. In an embodiment, the training data is used to find a relationship between the image data pixel intensities and spatial dose levels inside the target volumes.

In block 506, information specific to another (new) patient is input into the prediction model.

In block 508, the prediction model calculates a spatial dose distribution inside a target volume in the patient.

In block 510, a dose-volume histogram and associated three-dimensional dose distribution information are produced based on the spatial dose distribution. The dose-volume histogram and the three-dimensional dose distribution information can be subsequently used to develop a treatment plan that is specific to the patient. The radiation treatment plan tunes the radiation map for the target volume to the three-dimensional dose distribution information, so that a non-uniform dose distribution is applied to the target volume during radiation treatment.

In block 512, feedback from application of the treatment plan to the patient can be incorporated into the training data or the validation data, to refine or improve the prediction model.

In summary, embodiments according to the present invention use 3D images (e.g., PET images) as part of the input data, along with KBP data, to train and validate a prediction model, and also use the 3D images along with KBP in the prediction model to estimate the achievable dose distribution. The estimated dose distribution can be used to automatically provide spatially varying target dose levels (for dose painting).

Embodiments according to the present invention can be used to plan different types of external beam radiotherapy including, for example, IMRT, image-guided radiotherapy (IGRT), RapidArc^{™} radiotherapy, stereotactic body radiotherapy (SBRT), and stereotactic ablative radiotherapy (SABR).

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

The invention is defined in the claims as follows.

## Claims

1. A computer-implemented method (500), comprising:
accessing (506) information (301) that is specific to a patient;
determining (506) a prediction of a spatial dose distribution inside a target volume in the patient using the patient-specific information as an input to a prediction model (150), the prediction model established (504) using training data comprising data (202,204) resulting from applying other radiation treatment plans to other patients; and
producing (510) a dose-volume histogram and associated three-dimensional dose distribution information based on the prediction, the dose-volume histogram and the three-dimensional dose distribution information useful for developing a radiation treatment plan for the patient;
**characterized in that** the training data (202,204) comprises spatial distribution information indicating composition of target volumes in the other patients, and wherein the information indicating composition of target volumes is based on biological or chemical activity in the target volume.

2. The method of Claim 1, wherein the spatial distribution information comprises positron emission tomography, PET, image data and wherein the patient-specific information is selected from the group consisting of: size and shape of the target volume; size and shape of an organ at risk; type of an organ at risk; a part of the target volume that overlaps an organ; and a part of an organ that overlaps the target volume.

3. The method of Claim 1, further comprising using (504) the training data to find a relationship between image data pixel intensities of the spatial distribution information and spatial dose levels inside the target volumes.

4. The method of Claim 1, wherein the dose-volume histogram and the three-dimensional dose distribution information are also produced using the prediction model.

5. A computing system (100) comprising:
a central processing unit, CPU, (102) and
memory (104) coupled to the CPU and having stored therein instructions that, if executed by the computing system, cause the computing system to execute operations (500) comprising:
accessing (506) information (301) that is specific to a patient;
inputting (506) the patient-specific information into a prediction model, the prediction model established (504) using training data (202,204) comprising results from applying other radiation treatment plans to other patients;
calculating (508), with the prediction model, a spatial dose distribution inside a target volume in the patient; and
producing (510) a dose-volume histogram and associated three-dimensional dose distribution information based on the spatial dose distribution, the dose-volume histogram and the three-dimensional dose distribution information subsequently useful for developing and assessing a treatment plan that is specific to the patient;
**characterized in that** the training data (202,204) comprises spatial distribution information indicating composition of target volumes in the other patients, and wherein the information indicating composition of target volumes is based on biological or chemical activity in the target volume.

6. The system of Claim 5, wherein the spatial distribution information comprises positron emission tomography, PET, image data and wherein the patient-specific information is selected from the group consisting of: size and shape of the target volume; size and shape of an organ at risk; type of an organ at risk; a part of the target volume that overlaps an organ; and a part of an organ that overlaps the target volume.

7. The system of Claim 5, wherein the operations further comprise using (504) the training data to find a relationship between image data pixel intensities of the spatial distribution information and spatial dose levels inside the target volumes.

8. The system of Claim 5, wherein the radiation treatment plan tunes the three-dimensional dose distribution information to the target volume, wherein a non-uniform dose distribution is applied to the target volume in the patient during radiation treatment.

9. The system of Claim 5, wherein the operations further comprise incorporating feedback (512) from application of the radiation treatment plan into the prediction model.

10. A computer-readable storage medium (104) having computer-executable instructions for causing a computing system (104) to perform a method comprising:
accessing (502) a plurality of radiation treatment plans for a plurality of patients, the radiation treatment plans stored in a database in a knowledge-based planning system
generating a prediction model (150) using training data comprising results (202) from applying the radiation treatment plans to the patients;
inputting (506) information (301) specific to another patient into the prediction model;
calculating (508), with the prediction model, a spatial dose distribution inside a target volume in the patient; and
producing a dose-volume histogram and associated three-dimensional dose distribution information based on the spatial dose distribution, the dose-volume histogram and the three-dimensional dose distribution information subsequently useful for developing a treatment plan that is specific to the patient;
**characterized in that** the training data comprises spatial distribution information indicating composition of target volumes in the other patients, and wherein the information indicating composition of target volumes is based on biological or chemical activity in the target volume.

11. The computer-readable storage medium of Claim 10, wherein the spatial distribution information comprises positron emission tomography, PET, image data (204) and wherein the patient-specific information is selected from the group consisting of: size and shape of the target volume; size and shape of an organ at risk; type of an organ at risk; a part of the target volume that overlaps an organ; and a part of an organ that overlaps the target volume.

12. The computer-readable storage medium of Claim 10, wherein the method further comprises:
using (504) the training data to find a relationship between the image data pixel intensities of the spatial distribution information and spatial dose levels inside the target volumes; and
incorporating feedback from application of the radiation treatment plan into the prediction model.

13. The computer-readable storage medium of Claim 10, wherein the dose-volume histogram and the three-dimensional dose distribution information are also produced using the prediction model, and wherein the radiation treatment plan tunes the three-dimensional dose distribution information to the target volume.

## Patentansprüche

1. Computerimplementiertes Verfahren (500), umfassend:
Zugreifen (506) auf Informationen (301), die für einen Patienten spezifisch sind;
Bestimmen (506) einer Vorhersage einer räumlichen Dosisverteilung in einem Zielvolumen in dem Patienten unter Verwendung der patientenspezifischen Informationen als Eingabe in ein Vorhersagemodell (150), wobei das Vorhersagemodell unter Verwendung von Trainingsdaten erstellt wird (504), die Daten (202, 204) umfassen, die aus der Anwendung anderer Strahlenbehandlungspläne auf andere Patienten resultieren; und
Erzeugen (510) eines Dosis-Volumen-Histogramms und zugeordneter dreidimensionaler Dosisverteilungsinformationen auf Grundlage der Vorhersage, wobei das Dosis-Volumen-Histogramm und die dreidimensionalen Dosisverteilungsinformationen für die Entwicklung eines Strahlenbehandlungsplans für den Patienten nützlich sind;
**dadurch gekennzeichnet, dass** die Trainingsdaten (202, 204) räumliche Verteilungsinformationen umfassen, die die Zusammensetzung von Zielvolumen in den anderen Patienten anzeigen, und wobei die Informationen, die die Zusammensetzung von Zielvolumen anzeigen, auf biologischer oder chemischer Aktivität in dem Zielvolumen basieren.

2. Verfahren nach Anspruch 1, wobei die räumlichen Verteilungsinformationen Positronen-Emissions-Tomographie-(PET-)Bilddaten umfassen und wobei die patientenspezifischen Informationen ausgewählt sind aus der Gruppe bestehend aus:
Größe und Form des Zielvolumens; Größe und Form eines gefährdeten Organs; Art eines gefährdeten Organs; einem Teil des Zielvolumens, der ein Organ überlappt; und einem Teil eines Organs, der das Zielvolumen überlappt.

3. Verfahren nach Anspruch 1, ferner umfassend Verwenden (504) der Trainingsdaten, um eine Beziehung zwischen Bilddatenpixelintensitäten der räumlichen Verteilungsinformationen und räumlichen Dosisniveaus in den Zielvolumen zu ermitteln.

4. Verfahren nach Anspruch 1, wobei das Dosis-Volumen-Histogramm und die dreidimensionalen Dosisverteilungsinformationen ebenfalls unter Verwendung des Vorhersagemodells erzeugt werden.

5. Rechensystem (100), umfassend:
eine zentrale Verarbeitungseinheit (central processing unit - CPU) (102) und
Speicher (104), der an die CPU gekoppelt ist und darin gespeicherte Anweisungen aufweist, die bei Ausführung durch das Rechensystem das Rechensystem dazu veranlassen, Vorgänge (500) auszuführen, die Folgendes umfassen:
Zugreifen (506) von Informationen (301), die für einen Patienten spezifisch sind;
Eingeben (506) der patientenspezifischen Informationen in ein Vorhersagemodell, wobei das Vorhersagemodell unter Verwendung von Trainingsdaten (202, 204) erstellt wird (504), die Ergebnisse aus der Anwendung anderer Strahlenbehandlungspläne auf andere Patienten umfassen;
Berechnen (508), mit dem Vorhersagemodell, einer räumlichen Dosisverteilung in einem Zielvolumen in dem Patienten; und
Erzeugen (510) eines Dosis-Volumen-Histogramms und zugeordneter dreidimensionaler Dosisverteilungsinformationen auf Grundlage der räumlichen Dosisverteilung, wobei das Dosis-Volumen-Histogramm und die dreidimensionalen Dosisverteilungsinformationen anschließend für die Entwicklung und Bewertung eines Behandlungsplans, der für den Patienten spezifisch ist, nützlich sind;
**dadurch gekennzeichnet, dass** die Trainingsdaten (202, 204) räumliche Verteilungsinformationen umfassen, die die Zusammensetzung von Zielvolumen in den anderen Patienten anzeigen, und wobei die Informationen, die die Zusammensetzung von Zielvolumen anzeigen, auf biologischer oder chemischer Aktivität in dem Zielvolumen basieren.

6. System nach Anspruch 5, wobei die räumlichen Verteilungsinformationen Positronen-Emissions-Tomographie-(PET-)Bilddaten umfassen und wobei die patientenspezifischen Informationen ausgewählt sind aus der Gruppe bestehend aus:
Größe und Form des Zielvolumens; Größe und Form eines gefährdeten Organs; Art eines gefährdeten Organs; einem Teil des Zielvolumens, der ein Organ überlappt; und einem Teil eines Organs, der das Zielvolumen überlappt.

7. System nach Anspruch 5, wobei die Vorgänge ferner Verwenden (504) der Trainingsdaten, um eine Beziehung zwischen Bilddatenpixelintensitäten der räumlichen Verteilungsinformationen und räumlichen Dosisniveaus in den Zielvolumen zu ermitteln umfassen.

8. System nach Anspruch 5, wobei der Strahlenbehandlungsplan die dreidimensionalen Dosisverteilungsinformationen auf das Zielvolumen abstimmt, wobei eine nicht einheitliche Dosisverteilung während der Strahlbehandlung auf das Zielvolumen in dem Patienten angewendet wird.

9. System nach Anspruch 5, wobei die Vorgänge ferner Einbeziehen (512) von Feedback aus der Anwendung des Strahlenbehandlungsplans in das Vorhersagemodell umfassen.

10. Computerlesbares Speichermedium (104) mit computerausführbaren Anweisungen zum Veranlassen eines Rechensystems (104), ein Verfahren durchzuführen, das Folgendes umfasst:
Zugreifen (502) auf eine Vielzahl von Strahlenbehandlungsplänen für eine Vielzahl von Patienten, wobei die Strahlenbehandlungspläne in einer Datenbank in einem wissensbasierten Planungssystem gespeichert sind;
Erzeugen eines Vorhersagemodells (150) unter Verwendung von Trainingsdaten, die Ergebnisse (202) aus der Anwendung der Strahlenbehandlungspläne auf die Patienten umfassen;
Eingeben (506) von Informationen (301), die für einen anderen Patienten spezifisch sind, in das Vorhersagemodell;
Berechnen (508), mit dem Vorhersagemodell, einer räumlichen Dosisverteilung in einem Zielvolumen in dem Patienten; und
Erzeugen eines Dosis-Volumen-Histogramms und zugeordneter dreidimensionaler Dosisverteilungsinformationen auf Grundlage der räumlichen Dosisverteilung, wobei das Dosis-Volumen-Histogramm und die dreidimensionalen Dosisverteilungsinformationen anschließend für die Entwicklung eines Behandlungsplans, der für den Patienten spezifisch ist,
nützlich sind;
**dadurch gekennzeichnet, dass** die Trainingsdaten räumliche Verteilungsinformationen umfassen, die die Zusammensetzung von Zielvolumen in den anderen Patienten anzeigen, und wobei die Informationen, die die Zusammensetzung von Zielvolumen anzeigen,
auf biologischer oder chemischer Aktivität in dem Zielvolumen basieren.

11. Computerlesbares Speichermedium nach Anspruch 10, wobei die räumlichen Verteilungsdaten Positronen-Emissions-Tomographie-(PET-)Bilddaten (204) umfassen und wobei die patientenspezifischen Informationen ausgewählt sind aus der Gruppe bestehend aus: Größe und Form des Zielvolumens; Größe und Form eines gefährdeten Organs; Art eines gefährdeten Organs;
einem Teil des Zielvolumens, der ein Organ überlappt; und einem Teil eines Organs, der das Zielvolumen überlappt.

12. Computerlesbares Speichermedium nach Anspruch 10, wobei das Verfahren ferner Folgendes umfasst:
Verwenden (504) der Trainingsdaten, um eine Beziehung zwischen den Bilddatenpixelintensitäten der räumlichen Verteilungsinformationen und räumlichen Dosisniveaus in den Zielvolumen zu ermitteln; und
Einbeziehen von Feedback aus der Anwendung des Strahlenbehandlungsplans in das Vorhersagemodell.

13. Computerlesbares Speichermedium nach Anspruch 10, wobei das Dosis-Volumen-Histogramm und die dreidimensionalen Dosisverteilungsinformationen ebenfalls unter Verwendung des Vorhersagemodells erzeugt werden und wobei der Strahlenbehandlungsplan die dreidimensionalen Dosisverteilungsinformationen auf das Zielvolumen abstimmt.

## Revendications

1. Procédé mis en œuvre par ordinateur (500), comprenant :
l'accès (506) à des informations (301) qui sont spécifiques à un patient ;
la détermination (506) d'une prévention d'une distribution spatiale de dose à l'intérieur d'un volume cible chez le patient en utilisant les informations spécifiques au patient servant d'entrée dans un modèle de prévision (150), le modèle de prévision étant établi (504) en utilisant des données d'entraînement comprenant des données (202, 204) résultant de l'application d'autres plans de traitement par rayonnement à d'autres patients ; et
la production (510) d'un histogramme dose-volume et des informations de distribution de dose tridimensionnelles associées sur la base de la prévision, l'histogramme dose-volume et les informations de distribution de dose tridimensionnelles étant utiles pour le développement d'un plan de traitement par rayonnement destiné au patient ;
**caractérisé en ce que** les données d'entraînement (202, 204) comprennent des informations de distribution spatiale indiquant la composition des volumes cibles chez les autres patients, et dans lequel les informations indiquant la composition des volumes cibles sont basées sur l'activité biologique ou chimique dans le volume cible.

2. Procédé selon la revendication 1, dans lequel les informations de distribution spatiale comprennent des données d'image de tomographie par émission de positrons, TEP, et dans lequel les informations spécifiques au patient sont sélectionnées dans le groupe constitué par : la taille et la forme du volume cible ; la taille et la forme d'un organe à risque ; le type d'un organe à risque ; une partie du volume cible qui chevauche un organe ; et une partie d'un organe qui chevauche le volume cible.

3. Procédé selon la revendication 1, comprenant en outre l'utilisation (504) des données d'entraînement pour trouver une relation entre les intensités de pixel des données d'image des informations de distribution spatiale et les niveaux de dose spatiale à l'intérieur des volumes cibles.

4. Procédé selon la revendication 1, dans lequel l'histogramme dose-volume et les informations de distribution de dose tridimensionnelles sont également produits en utilisant le modèle de prévision.

5. Système informatique (100) comprenant :
une unité centrale de traitement, CPU, (102) et
une mémoire (104) couplée à la CPU et dans laquelle sont stockées des instructions qui, si elles sont exécutées par le système informatique, amènent le système informatique à exécuter des opérations (500) comprenant :
l'accès (506) à des informations (301) qui sont spécifiques à un patient ;
l'entrée (506) des informations spécifiques au patient dans un modèle de prévision, le modèle de prévision étant établi (504) en utilisant des données d'entraînement (202, 204) comprenant les résultats provenant de l'application d'autres plans de traitement par rayonnement à d'autres patients ;
le calcul (508), avec le modèle de prévision, d'une distribution spatiale de dose à l'intérieur d'un volume cible chez le patient ;
et
la production (510) d'un histogramme dose-volume et des informations de distribution de dose tridimensionnelles associées sur la base de la distribution spatiale de dose, l'histogramme dose-volume et les informations de distribution de dose tridimensionnelles étant utiles par la suite pour le développement et l'évaluation d'un plan de traitement qui est spécifique au patient ;
**caractérisé en ce que** les données d'entraînement (202, 204) comprennent des informations de distribution spatiale indiquant la composition des volumes cibles chez les autres patients, et dans lequel les informations indiquant la composition des volumes cibles sont basées sur l'activité biologique ou chimique dans le volume cible.

6. Système selon la revendication 5, dans lequel les informations de distribution spatiale comprennent des données d'image de tomographie par émission de positrons, TEP, et dans lequel les informations spécifiques au patient sont sélectionnées dans le groupe constitué par : la taille et la forme du volume cible ; la taille et la forme d'un organe à risque ; le type d'un organe à risque ; une partie du volume cible qui chevauche un organe ; et une partie d'un organe qui chevauche le volume cible.

7. Système selon la revendication 5, dans lequel les opérations comprennent en outre l'utilisation (504) des données d'entraînement pour trouver une relation entre les intensités de pixel des données d'image des informations de distribution spatiale et les niveaux de dose spatiale à l'intérieur des volumes cibles.

8. Système selon la revendication 5, dans lequel le plan de traitement par rayonnement accorde les informations de distribution de dose tridimensionnelles au volume cible, dans lequel une distribution de dose non uniforme est appliquée au volume cible chez le patient pendant le traitement par rayonnement.

9. Système selon la revendication 5, dans lequel les opérations comprennent en outre l'incorporation de la rétroaction (512) provenant de l'application du plan de traitement par rayonnement dans le modèle de prévision.

10. Support de stockage lisible par ordinateur (104) ayant des instructions exécutables par ordinateur destinées à amener un système informatique (104) à réaliser un procédé comprenant :
l'accès (502) à une pluralité de plans de traitement par rayonnement destinés à une pluralité de patients, les plans de traitement par rayonnement étant stockés dans une base de données dans un système de planification à base de connaissance la génération d'un modèle de prévision (150) en utilisant des données d'entraînement comprenant des résultats (202) provenant de l'application des plans de traitement par rayonnement aux patients ;
l'entrée (506) des informations (301) spécifiques à un autre patient dans le modèle de prévision ;
le calcule (508), avec le modèle de prévision, d'une distribution spatiale de dose à l'intérieur d'un volume cible chez le patient ; et
la production d'un histogramme dose-volume et des informations de distribution de dose tridimensionnelles associées sur la base de la distribution spatiale de dose, l'histogramme dose-volume et les informations de distribution de dose tridimensionnelles étant utiles par la suite pour le développement d'un plan de traitement qui est spécifique au patient ;
**caractérisé en ce que** les données d'entraînement comprennent des informations de distribution spatiale indiquant la composition des volumes cibles chez les autres patients, et dans lequel les informations indiquant la composition des volumes cibles sont basées sur l'activité biologique ou chimique dans le volume cible.

11. Support de stockage lisible par ordinateur selon la revendication 10, dans lequel les informations de distribution spatiale comprennent des données d'image de tomographie par émission de positons, TEP, (204) et dans lequel les informations spécifiques au patient sont sélectionnées dans le groupe constitué par : la taille et la forme du volume cible ; la taille et la forme d'un organe à risque ; le type d'un organe à risque ; une partie du volume cible qui chevauche un organe ; et une partie d'un organe qui chevauche le volume cible.

12. Support de stockage lisible par ordinateur selon la revendication 10, dans lequel le procédé comprend en outre :
l'utilisation (504) des données d'entraînement pour trouver une relation entre les intensités de pixel des données d'image des informations de distribution spatiale et les niveaux de dose spatiale à l'intérieur des volumes cibles ; et
l'incorporation de la rétroaction provenant de l'application du plan de traitement par rayonnement dans le modèle de prévision.

13. Support de stockage lisible par ordinateur selon la revendication 10, dans lequel l'histogramme dose-volume et les informations de distribution de dose tridimensionnelles sont également produits en utilisant le modèle de prévision, et dans lequel le plan de traitement par rayonnement accorde les informations de distribution de dose tridimensionnelles au volume cible.
